# EUROPEAN PATENT APPLICATION

(11) **EP 4 663 147 A1**
(43) Date of publication of application: **17.12.2025**
(21) Application number: 24315284.0
(22) Date of filing: 12.06.2024
(51) Int. Cl.: A61B 34/30, A61B 90/00, A61F 2/24, A61B 34/20

(54) **A MEDICAL DEVICE CONTROLLER AND MEDICAL SYSTEM COMPRISING THE DEVICE CONTROLLER**

(71) Applicant: Caranx Medical, 75013 Paris (FR)
(72) Inventor: The designation of the inventor has not yet been filed
(74) Representative: Hepp Wenger Ryffel AG

(57) **Abstract**

The invention relates to a medical device controller (101) for positioning at least one medical instrument (11, 12, 13) in proximity of an anatomic structure, in particular a valve. The medical device controller (101) comprises a control unit (2) which has an input interface (32) connected or connectable to an imaging source (3) for receiving imaging data (31), a robotic interface (41) connected or connectable to a robotic controller (4) for transmitting control commands (81) to the robotic controller (4), a processing unit (8) adapted for receiving imaging data (31) via the input interface (32) and adapted for transmitting at least one control command (81) via the robotic interface (41) to the robotic controller (4) for adjusting at least one medical instrument (11, 12, 13) to achieve a target mechanical characteristic (6), and drive control feedback means (7) configured for generating feedback data (72) indicative of a current mechanical characteristic (5) of the at least one medical instrument (11, 12, 13) with respect to the target mechanical characteristic (6) and transmitting the feedback data (72) to the processing unit (8). The control unit (2) has a feedback loop (71) established between the drive control feedback means (7) and the processing unit (8) which is configured to continuously update the at least one control command (81) in real-time based on the feedback data (72) to achieve the target mechanical characteristic (6).

## Description

The invention relates to a medical device controller and a medical system comprising the medical device controller for positioning at least one medical instrument in proximity of an anatomic structure, in particular a valve annulus.

Minimally invasive endovascular procedures, in particular heart valve implantations such as transcatheter aortic valve implantation / replacement (TAVI/TAVR), require accurate and consistent positional/orientational placement of implants. This poses a plurality of challenges for pre-operative planning of the procedure and the operation, and in particular for proper spatial configuration of the medical instrument(s), which may be subject to human operator limitations. In particular, due to inherent human sensory limitations and the time needed for receiving and interpreting data indicative of the position of the medical instrument(s) (e.g. imaging data or motor feedback data, to be conveyed to the operator and processed by the operator) precise, real-time optimization of medical instrument(s) positioning/orienting is often challenging.

This problem is even more apparent since clinicians have to rely on abstract data indicative of the position of the medical instrument(s), often times even from a plurality of different data sources, such as position data, imaging data, in particular CT and/or fluoroscopy data, force data, data indicative of a pose of the medical instrument(s), and/or a deployment status of an implant. In particular, the mutual dependency of instrument manipulations, e.g. positioning, orienting, deployment or pose adjustment, manipulating the medical instrument(s) based on this data can be exceedingly complex.

Moreover, based on these data, in particular from multiple sources, often times a plurality of manipulations has to be carried out by a clinician or a plurality of clinicians, such as simultaneous positioning of a plurality of medical instrument(s) and/or deployment of an implant during an intravascular procedure.

This may lead to a steep learning curve of clinicians, rendering the endovascular procedures dependent on the skills of a clinician and requiring training time to effectively perform the procedures. Moreover, the procedure is limited by a perception-cognition-action loop of the clinician for spatially configurating the medical instrument(s).

Even minor malpositioning/malorienting/maldeployment of the medical instrument(s)/implant(s) may have detrimental effects and surgical risks which can result in stroke/thromboembolism, aortic dissection, infection, hematoma, blood clots, and/or valve regurgitation/migration, and can even lead to fatal surgical outcomes.

CN 215458616 U discloses a surgical robot system which comprises a remotely controlled, mobile support structure, a rotating table with an end effector for valve stent implantation, and a driving assembly for precise movement and rotation, aimed at reducing a clinician's radiation exposure during operations.

However, the prior art lacks a solution which enables reliable and exact adaptation of the mechanical characteristic of one or more medical instruments. In particular, the prior art lacks a medical device controller which enables complex manipulation of one or more medical instruments to achieve a target mechanical characteristic based on imaging data and/or other data sources in a partially or fully autonomous manner.

In particular, accurate placement of one or more medical instruments comprising a heart valve prosthesis during TAVI/TAVR would be beneficial since an optimal implantation depth, within less than 6 mm below the annulus plane, is currently only achieved for less than half of the patients (e.g. 43.2 % of the patient according to Urena M, Webb JG, Tamburino C, et al. Optimal Implantation Depth and Adherence to Guidelines on Permanent Pacing to Improve the Results of Transcatheter Aortic Valve Replacement With the Medtronic CoreValve System: The CoreValve Prospective, International, Post-Market ADVANCE-II Study. JACC Cardiovasc Interv. 2015;8 (6) :837-846).

It is an object of the present invention to overcome these and other disadvantages of the prior art and provide a medical device controller and medical system comprising the medical device controller which solves the above-mentioned technical problems, and which in particular allows a reliable spatial positioning and deployment of an implant/mechanical device. This object is solved by a medical device controller and medical system according to the independent claims. Further embodiments result from the dependent claims.

The medical device controller is adapted for positioning at least one medical instrument in proximity of an anatomic structure, in particular a valve annulus. The medical device controller comprises a control unit which has an input interface connected or connectable to an imaging source for receiving imaging data, in particular processed imaging data. The controller comprises a robotic interface connected or connectable to a robotic controller for transmitting control commands to the robotic controller. The controller further comprises a processing unit adapted for receiving imaging data via the input interface and is adapted for transmitting at least one control command via the robotic interface to the robotic controller for adjusting at least one medical instrument to achieve a target mechanical characteristic. The controller comprises drive control feedback means which are configured for generating feedback data indicative of a current mechanical characteristic of the at least one medical instrument with respect to the target mechanical characteristic and transmitting the feedback data to the processing unit. The control unit has a feedback loop established between the drive control feedback means and the processing unit. The feedback loop is configured to continuously update the at least one control command in real-time based on the feedback data to achieve the target mechanical characteristic.

The current/target mechanical characteristic may comprise or consist of a current/target position, shape, orientation, a deployment status of the medical instrument, and/or a force value exerted onto the medical instrument. The current/target mechanical characteristic may be a spatial configuration, i.e. a current/target position, shape, orientation and/or deployment status of the medical instrument. Adjusting the medical instrument from demonstrating the current mechanical characteristic to the medical instrument achieving the target mechanical characteristic may be achieved by moving and/or adjustment of the orientation and/or force of the medical instrument. The target mechanical characteristic may be determined based on pre-operative planning.

The feedback loop may be a closed feedback loop which uses self-regulating processes that minimizes discrepancies based on the feedback data and automated corrective control commands. It may, however, also receive instructions from an operator.

The medical device controller may be adapted for carrying out pre-operative planning to determine the target mechanical characteristic or the input interface may be adapted to receive the target mechanical characteristic.

Additionally, the generated feedback data may be further indicative of relevant internal states and/or characteristics of the robotic controller, such as actuating speed, heat, and/or slippage between the at least one medical instrument and the vasculature (e.g. improper placement of the heart valve prosthesis in proximity of the aortic annulus).

Alternatively or additionally, the processing unit may be adapted for generating and transmitting a control command such that a predetermined force value or a force value in a predetermined force value range is achieved. The drive control feedback means may then be configured for generating feedback data indicative of the current mechanical characteristic and/or a current force value of the at least one medical instrument with respect to the predetermined target force value or the force value of the predetermined force value range. The control unit may have a feedback loop established between the drive control feedback means and the processing unit which is configured to continuously update the at least one control command in real-time based on the feedback data to reach the target force value or the force value of the predetermined force value range.

The force value/current force value/predetermined force value may be indicative of a force exerted on the at least one medical instrument. The current force value may be determined by the robotic controller, e.g. via a force sensor such as a load cell using strain gauges or the piezoelectric effect, which measures the force required to move the at least one medical instrument. This force value may be transmitted from the robotic controller to the processing unit continuously.

The drive control feedback means may be configured for generating a first feedback data indicative of a first current mechanical characteristic of a first medical instrument, in particular a guidewire, with respect to a first target mechanical characteristic. The control unit may have a first feedback loop established between the drive control feedback means and the processing unit which is configured to continuously update a first control command in real-time based on the first feedback data to achieve the first target mechanical characteristic of the first medical instrument.

This may provide a reliable controller for achieving a first target mechanical characteristic of the first medical instrument, e.g. a guidewire/catheter, in particular a pre-shaped guidewire/catheter such as a SAFARI guidewire/catheter, within the left ventricle or in proximity of the aortic valve annulus, in real-time.

The drive control feedback means may be configured for generating second feedback data indicative of a second current mechanical characteristic of a second medical instrument, in particular a heart valve prosthesis or a delivery device, with respect to a target mechanical characteristic. The second current mechanical characteristic is preferably different from the first current mechanical characteristic. The control unit may have a second feedback loop established between the drive control feedback means and the processing unit which is configured to continuously update a second control command to achieve the second target mechanical characteristic of the second medical instrument.

This may provide a reliable controller for achieving a second target mechanical characteristic of the second medical instrument, e.g. the heart valve prosthesis or the delivery device of the heart valve prosthesis. The second target mechanical characteristic may be a position within the left ventricle of the heart, the heart annulus, in particular the aortic heart annulus, the aortic arch, the coronary ostia, a target implantation depth of the heart valve prosthesis, or a deployment status of the heart valve prosthesis, preferably a fully deployed status of the heart valve prosthesis.

In particular, a medical device controller which is adapted for achieving the first and the second target mechanical characteristic may achieve reliable positioning/orienting/deployment of a plurality of medical instruments at different target mechanical characteristics. Human perception is often limited to the manipulation of one complex task such as manipulation of the first medical instrument. These limitations can be overcome with the present invention, by prioritizing stimuli and avoiding sensory overload.

The controller according to the invention may further reduce the necessity of a plurality of clinicians manipulating the different medical instruments and simultaneously facilitate mutual synchronization while manipulating the medical instruments. A common feedback loop can be provided to generate multimodal control of the first and second control commands, instead of separate first and second feedback loops. A plurality of feedback loops or a common feedback loop may be established between the processing unit and the driver control feedback means for more than two, in particular more than three, preferably more than four medical instruments, to continuously update control commands in real-time based on the feedback data to achieve the target mechanical characteristic.

The drive control feedback means may be configured for generating feedback data based on the imaging data, in particular fluoroscopy and/or computer tomography (CT) imaging data, from the imaging source indicative of the current mechanical characteristic of the at least one medical instrument and the target mechanical characteristic of the at least one medical instrument, in particular with respect to the anatomic structure of the patient.

The anatomic structure may be the aortic wall, the aortic root, the aortic arch, the native valve annulus, in particular the aortic valve annulus, the aortic valve cusps, in particular the non-coronary cusp, or the left ventricle, in particular the apex of the left ventricle.

Using imaging data for determining the target and current mechanical characteristic of the medical instruments, in particular of the first and second medical instrument, may provide increased precision, patient safety, and facilitate complex medical procedures such as TAVI/TAVR and in turn improve surgical outcomes. The imaging data normally has to be analysed in real-time during a surgical procedure such that a clinician may have to take decisions concerning the current and target mechanical characteristic based on the imaging data in very short time frames. Hence, the analysis can be considerably limited by the cognition-perception loop. The controller may be adapted for reliably determining these mechanical characteristic(s) in real-time, in particular for a plurality of medical instruments, via computer vision, e.g. via machine learning algorithms.

The medical device controller may be specifically adapted to also or alternatively include prior imaging data indicative of a prior mechanical characteristic of the medical instrument(s) detected before the point in time of detecting the imaging data to determine the feedback data.

A clinician may manipulate the medical instrument(s) only based on the currently displayed imaging data, e.g. fluoroscopy imaging data. The medical device controller may enable a more informed decision of the current and target mechanical characteristic based on including prior imaging data while determining the feedback data.

The feedback loop may be configured to determine a position of at least one of the coronary ostia, in particular both coronary ostia. The feedback loop may further be configured to determine if the heart valve prosthesis achieving the target mechanical characteristic would obstruct the coronary ostium and, if the coronary ostium would be obstructed, modify the target mechanical characteristic such that the coronary ostium is not obstructed.

The heart valve prosthesis in the target mechanical characteristic can assume a fully deployed state positioned for replacing a native valve or a subsection of the native valve such that it does not obstruct the coronary ostia.

This may provide an additional constraint for determining the target mechanical characteristic such as to avoid obstruction of the coronary ostium which may otherwise be difficult to assess from a clinician's perspective, i.e. because the heart valve prosthesis is only visible in its collapsed state in the imaging data and/or the coronary ostia positions/orientations are difficult to clearly identify in the imaging data, in particular in the 2D fluoroscopy data.

The imaging data may only display the heart valve prosthesis in the collapsed state, such that positioning of the heart valve prosthesis with respect to the coronary ostia may be difficult to assess visually based on the imaging data. The medical device controller may be adapted to detect visual markers of the medical instruments, in particular the heart valve prosthesis or a catheter carrying the heart valve prosthesis, which are indicative of the three-dimensional orientation with respect to the anatomic structure, in particular the coronary ostia.

The feedback loop may be based on feedback data including a size, a geometry, and/or a target implantation depth of a heart valve prosthesis of the at least one medical instrument to further refine the at least one medical instrument achieving the target mechanical characteristic. These parameters may be input via an input interface, in particular by a graphical user interface by a clinician. The target implantation depth is defined as the distance between the valve annulus to the tip of the heart valve prosthesis and may e.g. be input in percent of the total length of the heart valve prosthesis.

The at least one control command may comprise a movement control command for at least one driver of the robotic controller for longitudinal translation of the at least one medical instrument, for rotating the at least one medical instrument around a longitudinal axis of the at least one medical instrument, and/or for tilting the at least one medical instrument with respect to the longitudinal axis.

This may allow a reliable movement of the medical instrument(s) demonstrating the current mechanical characteristic to achieve the target mechanical characteristic, e.g. for positioning the heart valve prosthesis at an implantation depth in proximity of the heart valve annulus, in particular the aortic valve annulus.

The movement control command for longitudinal translation, rotating, and/or tilting of the at least one medical instrument may be based on independent feedback loops between the control unit and the motor control feedback means.

The term longitudinal axis within the context of this application may relate to a curvilinear longitudinal axis, if e.g. the medical instrument is deflected to a bent shape.

The movement control command for at least one driver of the robotic controller may also be configured for changing a shape of the at least one medical instrument, in particular to a predetermined shape, e.g. deflect a distal tip to a predetermined bent shape via tensioning an actuating element.

The movement control command for at least one driver of the robotic controller may also be configured for changing the configuration of at least one moveable element within at least one medical instrument. The movement control command may be adapted to configure the medical instrument for or during a medical procedural step such as the deployment of the heart valve prosthesis.

The movement control command may be configured to operate the robotic controller to translate a distal cover or sheath for partially uncovering a heart valve prosthesis via tensioning an actuating element such as a pull wire. Alternatively, the movement control command may be configured to operate the robotic controller to translate a balloon element of the balloon catheter with respect to the at least one medical instrument, in particular to a delivery device such as a catheter.

The movement control command for at least one driver of the robotic controller may be configured for moving the first medical instrument or the second medical instrument in a previously described manner with respect to the other of the first medical instrument and the second medical instrument which optionally remains immovable. A plurality of different movement commands may be configured for moving at least a first and second medical instrument which are concentrically arranged with respect to each other. The movement commands may be configured for moving at least three medical instruments which are concentrically arranged with respect to each other, e.g. a heart valve prosthesis, a balloon catheter, a guidewire, and preferably a catheter sheath.

The at least one control command may comprise a deployment control command for deploying the medical instrument formed by a heart valve prosthesis. The drive control feedback means may be configured for generating feedback data indicative of a deployment status of the heart valve prosthesis. The feedback data may be based on a deployment status of a self-expanding heart valve prosthesis, or on a pressure value, preferably an input pressure value or a pressure value measured in a balloon catheter for deploying the heart valve prosthesis. The feedback data may be based on a volume value of a balloon catheter for deploying the heart valve prosthesis. The feedback data may be based on a relative volume with respect to a maximum volume of the balloon catheter for deploying the heart valve prosthesis.

Deploying the medical instrument in this manner enables for including additional information indicative of the deployment status which is normally not easily assessed by a clinician merely based on the imaging data, e.g. due to the accurate vasculature only being visible upon contrast agent administration. This may further prevent vascular injury, in particular vascular dissection or perforation and prevent dislodgement of calcifications, under-expansion and/or over-expansion of the heart valve prosthesis, and/or a post implantation movement of the heart valve prosthesis due to malpositioning.

The feedback data indicative of the deployment status may be determined by the robotic controller and transmitted to the processing unit of the medical device controller.

The medical device controller may be adapted to process fluoroscopy imaging data received by the input interface to identify inflation states of the balloon, interpret them, and generate feedback data and a control command based on the feedback data via the control unit. This control command may be adapted for operating the fluid inflation unit when transmitted to the robotic controller. The control unit may be adapted for generating a control command comprising halt/stop control command for halting the inflation via the fluid inflation unit depending on an identified unexpected inflation state. For example, the medical device controller may visually identify an unexpected balloon shape and then provide a halt or stop command based on a potential hazard.

The deployment status indicative of a self-expanding heart valve prosthesis may be determined based on movement of at least one medical instrument, e.g. retraction, of at least a first medical instrument, in particular a catheter sheath at least partially covering the self-expandable heart valve prosthesis, with respect to the second medical instrument formed by the self-expandable heart valve prosthesis.

The deployment status indicative of a self-expanding heart valve prosthesis may also be a cross-sectional dimension of the heart valve prosthesis determined based on the imaging data, in particular fluoroscopy, preferably by using computer vision. This may e.g. be achieved by the heart valve prosthesis being radio-paque and/or the administration of contrast agent for identifying the cross-sectional dimension of the vasculature.

In one embodiment, the movement control command(s) may be adapted for adjusting the at least one medical instrument in at least one translational degrees of freedom to achieve the target mechanical characteristic. Additionally or alternatively, the control command(s) may be adapted for adjusting the at least one medical instrument in at least one rotational degree of freedom, in particular two rotational degrees of freedom, to achieve the target mechanical characteristic.

The feedback loop of the control unit may be configured to continuously update the control command, in particular for longitudinal translation, and the deployment control command in a synchronized manner during deployment of the heart valve prosthesis.

This enables to provide a controller which may counteract displacement of the heart valve prosthesis occurring during the deployment of the heart valve prosthesis. Synchronizing the movement and deployment control command may allow to compensate the effects by blood flow and/or non-linear deployment of the heart valve prosthesis on the movement of the delivery device or of the heart valve prosthesis.

The heart valve prosthesis and/or the delivery device may be inadvertently positioned displaced with respect to a centerline of the vasculature pre-deployment. The movement control command(s) may be configured for maintaining the central position with respect to the surrounding vasculature, i.e. within the aortic annulus, parallel to the centerline prior and/or during the deployment based on the deployment control command(s). This position may also be defined as an intermediate target mechanical characteristic in which the heart valve prosthesis is in a non-deployed or not fully deployed state and the delivery device is positioned in the central position parallel to the centerline.

During deployment of the heart valve prosthesis, e.g. via a balloon catheter of the delivery device, the heart valve prosthesis/delivery device may be displaced with respect to the center position and parallel arrangement. The deployment may be affected by a non-homogenous blood flow distribution, a change in the blood flow, or non-linear deployment of the heart valve prosthesis/balloon catheter, which may be compensated for by the synchronized control commands. The control commands may be adapted for achieving the target mechanical characteristic with or without rapid pacing of the heart. The control commands may be adapted for achieving the target mechanical characteristic with or without administering contrast agent.

The robotic interface may be adapted for receiving sensor data, in particular from the robotic controller, and the drive control feedback means may be adapted for generating the feedback data based on the received sensor data.

This may enable a more targeted determination of the control command for the medical instrument(s) based on sensor data. The sensor data may be used in combination with the imaging data to achieve an even more accurate control command to achieve the target mechanical characteristic.

The sensor data may comprise or consist of force data indicative of a force exerted on the at least one medical instrument received from the robotic controller. The sensor data may comprise or consist of data indicative of a deployment status of the heart valve prosthesis. The sensor data may comprise or consist of data indicative of a current mechanical characteristic of the at least one medical instrument. The sensor data may comprise or consist of cardiac activity data indicative of a cardiac rhythm and/or respiratory activity data indicative of a respiratory rhythm of the patient.

The current mechanical characteristic of the at least one medical instrument may be detected via encoders and/or potentiometers, e.g. of the robotic controller, to indicate motor positions and/or positions of the medical instrument(s).

The cardiac activity data may be cardiac electrical activity data detected via an electrocardiogram (ECG), in particular an intravascular ECG, data of a blood pressure monitor, and/or data of a cardiac telemetry device. The respiratory activity data may be detected via a respiratory rate monitor and/or a spirometer.

The sensor data selected from at least one of the above, in particular in addition to the imaging data, enable generating more refined feedback data and, thereby also a more refined continuously updated control command to achieve the target mechanical characteristic.

The drive control feedback means may be configured for generating feedback data based on a plurality of input data from different sources, in particular selected from the previously described imaging data of the imaging source and from the previously described sensor data from the robotic controller. The drive control feedback means may be configured for applying a sensor fusion algorithm, in particular a Kalman Filter algorithm, to the plurality of input data to generate the feedback data.

This may enable to extract the essential meaningful data for determining a reliable control command. The control unit can be configured to assess the quality of the data so that the data from the various sources influence the calculated control command to varying degrees.

In a simple design, the algorithm for converting the input data from different sources to a control command may be Proportional Integral Derivate (PID) algorithm. In this manner the varying dynamics of the current and prior mechanical characteristics can be accounted for and disturbances and changes to the medical instrument(s) to achieve the target mechanical characteristic can be compensated in a simple manner in real-time.

Advantageously the medical device controller, in particular the control unit, may include a behavior model for predicting a behavior of the at least one medical instrument with respect to its surroundings, e.g. the vasculature surrounding it.

The behavior model and the input data may be used together with the control output to create a state observer. This state observer may allow to continuously update the control command of the system without using sensors. Advantageously, this can be used to speed up the feedback loop, and to decrease the frequency at which data needs to be acquired from X-ray exposure, e.g. by fluoroscopy, or slow imaging data/sensor data stacks.

The behavior model and the input data may be used together to compute the system's inverse kinematics, either directly or through an optimization algorithm. This enables to apply inverse model-based controls offering a high precision and accuracy.

The behavior model and the input data may be used together as a predictor of future mechanical characteristics. This predictor may in turn enable to apply model predictive control algorithms.

The controller, in particular the control unit, may be adapted to save processed feedback data or the input data, e.g. the imaging data and/or sensor data, to a memory, e.g. for reusing the saved data saved for prior mechanical characteristics for determining control commands to achieve the target mechanical characteristic.

The behavior model may be a machine learning algorithm and modelled by a deep neural network. The behaviour model may be adapted to be enhanced by learning residual dynamics, e.g. high-order interactions, which may be integrated in a non-linear manner in the feedback loop.

For a multimodal design, a neural network, preferably a convolutional neural network, in particular based on reinforcement learning, may alternatively be used for determining and continuously updating the control commands.

The control unit may be adapted for determining if the control command to achieve the target mechanical characteristic would result in a motor controller being controlled such as to exceed a force limit of the at least one medical instrument interacting with the anatomy of the patient. If it is detected that the force limit is exceeded, the control unit may be configured for adapting the control command such that the force limit is not exceeded. The force limit may be one of (i) a preset fixed force limit, (ii) a dynamic force limit determined based on the current mechanical characteristic of the at least one medical instrument with respect to an anatomic structure of the patient, in particular the left ventricle, and/or (iii) a dynamic force limit determined based on an angle of incidence of a longitudinal axis of the medical instrument interacting with the anatomic structure of the patient.

This enables to introduce constraints in the form of limiting forces such that certain anatomic regions or demonstrating certain mechanical characteristics may be avoided and patient safety can be optimized by e.g. avoiding perforation, pericardial bleeding, or even ventricular rupture which may result in serious medical complications.

The above-mentioned force limit may in particular be used for the medical instrument formed by a guidewire such that reliable and atraumatic placement by achieving the target mechanical characteristic, e.g. by positioning in the left ventricle, preferably in the left ventricle apex, of the patient can be achieved. In addition, the current mechanical characteristic of the guidewire in the form of a path of the guidewire in the aortic arch/ascending aorta and/or a change of a shape of the guidewire, e.g. a safari pre-shaped guidewire, in the left ventricle, preferably the native apex, due to compression may be detected based on the imaging data.

The current and/or target mechanical characteristic of the at least one medical instrument with respect to a patient anatomy may be (i) a position, a shape, and/or an orientation of a first medical instrument formed by a guidewire or catheter with respect to a native valve annulus or a native apex of the left ventricle or a force acting on the first medical instrument. In alternative or addition, the current and target mechanical characteristic may be (ii) a position, a shape, and/or an orientation of a second medical instrument formed by a heart valve prosthesis or a delivery device thereof with respect to a native valve annulus or a force acting on the second medical instrument. In alternative or addition, the current and target mechanical characteristic may be (iii) a deployment status of a medical instrument, in particular the heart valve prosthesis and/or a balloon catheter.

The guidewire or catheter may be a pre-shaped guidewire or catheter, in particular a pigtail guidewire or catheter.

In a preferred embodiment, all these current and target mechanical characteristics are processed by a common feedback loop or independent feedback loops to generate control commands.

This may enable reliable positioning/orienting/deployment of the guidewire or a catheter, the heart valve prosthesis, and/or the balloon catheter in a reliable manner, in particular in a synchronized manner with respect to each other.

The control unit may be adapted for generating a planned trajectory of the at least one medical instrument, in particular a delivery device, the planned trajectory extending from the at least one medical instrument demonstrating the current mechanical characteristic to the at least one medical instrument achieving the target mechanical characteristic. The planned trajectory may be generated via a trajectory generation algorithm based on a model of the anatomic structure of the patient received via the input interface. The model of the anatomic structure is preferably a pre-operative model of the anatomic structure of the patient, in particular a CT model of the aorta and the cardiac region.

This may enable a streamlined and precise navigation of the medical instrument(s) from demonstrating the current mechanical characteristic to achieving the target mechanical characteristic, in particular if the model, such as a CT model, is received via the input interface in conjunction with intra-operative imaging data, such as fluoroscopy data.

The trajectory generation algorithm may be selected from at least one of (i) a Dijkstra algorithm, (ii) a look-ahead tree search algorithm, (iii) a probabilistic roadmap, (iv) rapidly exploring random trees, and (v) a centerline algorithm. The trajectory generation algorithm is preferably adapted to generate a plurality of waypoints.

A look-ahead tree search algorithm may allow to take into consideration feasible configurations of the medical instruments, in particular the delivery device, the guidewire, and/or the heart valve prosthesis, to determine the planned trajectory.

The control unit may be adapted for determining or receiving a centerline with respect to an aorta wall of the aorta model. The control unit may be adapted for generating the planned trajectory by minimizing the distance of the at least one medical instrument to the centerline and/or by following the plurality of waypoints.

The plurality of waypoints may serve as intermediate target mechanical characteristic(s) which may facilitate optimizing the planned trajectory by optimizing subsections of the implantation path independently from each other, e.g. until achieving the final target mechanical characteristic at the native heart valve.

By minimizing the distance of the medical instrument(s) to the centerline, the risk of potential dislodgement of vascular plaque, e.g. calcifications, and/or the risk of tissue trauma can be minimized. At the same time, these models may reduce the computational complexity and thus real-time computation of the algorithms by defining waypoints along the centerline.

The medical device controller may be adapted for displaying the imaging data of the imaging source, and in particular an information indicative of a relation between the current mechanical characteristic and the target mechanical characteristic, in particular the at least one medical instrument demonstrating/achieving the current/target mechanical characteristic.

This may provide the clinician with further valuable information in the form of pre-processed data, effectively minimizing cognitive overload, such that clinicians are able to focus on salient medical evaluations and decision-making processes.

The medical device controller may be operable at least in (a) feedback control mode in which the feedback loop is configured to continuously update the at least one control command in real-time based on the feedback data to achieve the target mechanical characteristic, and (b) a tele-operative control mode, in which the device controller is adapted to receive input commands from a user and generate the control command, in particular the movement and/or deployment control command, via the control unit based on the input commands instead of the feedback loop. The medical device controller may have a user interface, in particular a graphical user interface, adapted to receive user instructions to switch between the feedback control mode and the tele-operative control mode. Alternatively, a switch also might be automatically triggered based on certain events.

This provides a reliable safety feature such that a clinician can always take over and tele-operatively operate the medical device controller to manipulate the medical instruments, such that the medical procedure can be reliably carried out in case of an error and/or malfunction. Moreover, the clinician can also always oversee the medical procedure and step in, in case of unforeseen events.

The medical device controller may comprise a user interface for transmitting input commands tele-operatively to the device controller, in particular a graphical user interface and/or a gamepad console or custom joystick interface. In addition, the medical device controller may have haptic binary input/output elements such as buttons. The medical device controller may further comprise signaling elements such as LEDs for signaling a status of the medical device controller, such as if the device controller currently is in the feedback or tele-operative control mode.

The medical device controller may be operable in an automated inflation mode and/or a user input inflation mode. In the automated inflation mode, the deployment control command is updated in an automated manner by at least one of (i) a predetermined pressure or volume limit for a safe operation of the balloon catheter, (ii) a predetermined inflation time of the balloon catheter to achieve the deployment of the heart valve prosthesis, and (iii) a predetermined temporal pressure or volume function to apply pressure or supply a fluid volume to the balloon catheter. In the user inflation mode, the deployment control command is updated by a user input via a user interface. In this case, the user input may be indicative of (i) a pressure or volume limit for a safe operation of the balloon catheter, (ii) an inflation time of the balloon catheter to achieve the deployment of the heart valve prosthesis, and/or (iii) a temporal pressure or volume function to apply pressure or supply a fluid volume to the balloon catheter.

This may provide a controlled inflation of the balloon catheter having regard to the pressure/volume when deploying the heart valve prosthesis. The inflation modes further provide an enhanced patient safety by reducing the risk of vessel damage while simultaneously ensuring reliable deployment of the heart valve prosthesis to a sufficient degree to prevent valve migration. Moreover, these inflation modes may provide means for optimizing inflation to patient-specific shapes and/or sizes of the anatomic structure, such as the aortic valve annulus, and/or for the selected heart valve prosthesis/balloon catheter.

A reliable time-based injection may be achieved by position control and/or speed control of an injection device injecting the fluid. This may be achieved by updating the control command based on the predetermined temporal pressure or volume function. This may allow controlling a syringe plunger of a syringe within predefined safe pressure limits. The robotic control may be adapted to be connectable to the syringe and may have the driver for controlling the position and/or speed of injection.

In particular the (predetermined) temporal pressure or volume function to apply pressure or supply fluid volume to the balloon catheter may optimize non-linear effects during valve deployment by varying the applied pressure/supplied fluid volume in accordance with specific states of deployment of the heart valve prosthesis. This may further improve procedural efficiency, resulting in expedited and safer minimally invasive surgical procedures.

The control unit and/or the robotic controller may be adapted to identify a prefilled volume of a fluid inflation unit, in particular the syringe. This enables to identify the prefilled volume after the fluid inflation unit was attached and installed in the robotic controller/the medical system.

The syringe with the prefilled volume may be installed with a fully retracted plunger in a locked position within the robotic controller/medical system. The syringe may be sealed in an airtight manner such that a negative pressure/partial vacuum is achieved in an interior spacing of the syringe, i.e. essentially no residual air is within the interior spacing.

The fluid inflation unit may have a pressure sensor, in particular a pressure sensor connected to the syringe outlet.

The outlet may be closable, e.g. via a valve or stopcock of the fluid inflation unit, if the pressure sensor detects a pressure value below and/or above a pressure threshold. In particular, an increase in pressure indicating a completed inflation of the heart valve prosthesis in the anatomy may be a pressure threshold.

The medical system/robotic controller and the syringe may be adapted to advance the plunger forward until an increase in pressure, i.e. a pressure threshold, is observed.

The medical system/robotic controller and the syringe may be adapted to subsequently retract the plunger again.

The medical system/robotic controller may be adapted to repeatedly advance and retract the plunger a plurality of times to identify an equilibrium/zero pressure point with a greater accuracy. Based on a known plunger position with respect to a known syringe length and cross section combined with the identified equilibrium/zero pressure point the medical system/robotic controller may be adapted to determine the prefilled volume of said syringe.

The control unit may be adapted to fully autonomously update the control command to retain the at least one medical instrument in a specific anatomic structure. The specific anatomic structure may be selected from (i) the left ventricle and/or the aortic annulus, preferably maintaining contact with or in the vicinity of the apex of the left ventricle, or (ii) the non-coronary cusp, and/or the at least one medical instrument achieving the target mechanical characteristic.

In alternative or addition, the control unit may be adapted to provide a warning signal to a user, in particular on a graphical and/or acoustic user interface, if the medical instrument is not retained in the specific anatomic structure.

This may provide a control unit for consistently retaining the at least one medical instrument, in particular the guidewire and/or the heart valve prosthesis in a particular anatomic structure, in particular the left ventricle or apex of the left ventricle for the guidewire and the aortic annulus or a specific implantation depth or orientation for the heart valve prosthesis.

The at least one medical instrument which shall be kept in the left ventricle and/or the aortic annulus may be a guidewire, preferably having a pre-shaped tip, in particular an intraventricular pacing guidewire, e.g. an intraventricular SAFARI pacing guidewire.

The at least one medical instrument which shall be kept in the non-coronary cusp or achieving the target mechanical characteristic may be a catheter/guidewire, in particular a pre-shaped catheter/guidewire, preferably a pigtail catheter/guidewire.

The at least one feedback loop, in particular all feedback loops, established between the drive control means and the processing unit for continuously updating the control command can be based on at least one of (i) a linear feedback control mode, (ii) a proportional-integral-derivative feedback control mode, (iii) an optimization-based control mode based on the feedback data and a set of constraints, and/or (iv) a neural network feedback control mode. The constraints may be defined by (I) the anatomic structure constraints, (II) operational constraints, and/or (III) physical or safely limit constraints.

These feedback loops enable robust means for the feedback loop which can be implemented in real-time.

The optimization-based control mode may be adapted to compute an optimization algorithm.

The feedback loop established between the drive control feedback means and the processing unit may be configured for continuously updating at least a first control command and a second control command for different actuators of the robotic controller to achieve the target mechanical characteristic. The feedback loop may be used to actuate different actuators to adjust the at least one medical instrument demonstrating the current mechanical characteristic towards the at least one medical instrument achieving the target mechanical characteristic by using the optimization algorithm. The optimization algorithm may further comprise determining at least first and second control commands for at least two different actuators which minimize a difference between the at least one medical instrument demonstrating the current mechanical characteristic and the at least one medical instrument achieving the target mechanical characteristic.

By taking the actuations and the change of the current mechanical characteristic of the at least one medical instrument into consideration, the optimization algorithm can be configured for also including the constraints of the environment, e.g. the patient's vascular anatomy, and constraints of the robotic controller and the at least one medical instrument, e.g. the shape of the at least one medical instrument or forces exerted on it.

This feedback loop may include the step of (i) updating a stiffness matrix of known positions of the at least one medical instrument, (ii) computing a Jacobian matrix which relates the actuation of the different drivers to a change of the at least one medical instrument demonstrating the current mechanical characteristic towards the at least one medical instrument achieving the target mechanical characteristic, and (ii) deriving a compliance matrix based on the stiffness matrix and the inverse Jacobian matrix indicative of how actuation of the different drivers affects the medical instrument. This feedback loop further includes the steps of (iv) computing actuations of the different drivers to achieve the target mechanical characteristic while minimizing the difference between the at least one medical instrument demonstrating the current mechanical characteristic and the at least one medical instrument achieving the target mechanical characteristic and further (v) determining the first and second control command for the at least two different drivers based on the computed actuations.

Such a feedback loop allows for effective determination of first and second control commands for independent drivers which minimize the differences between the at least one medical instrument demonstrating the current mechanical characteristic and the at least one medical instrument achieving the target mechanical characteristic.

Another aspect of the present invention relates to a medical system comprising a medical device controller as previously described and a robotic controller having a plurality of drivers for moving the at least one medical instrument.

This facilitates the provision of a synchronized medical system comprising a medical device controller and a robotic controller, engineered to achieve seamless compatibility and optimal coordination. This may be particularly advantageous, since the feedback loop can be specifically tuned, in particular trained via training deep neural networks, to optimize the control commands in a reliable manner for use with the robotic controller.

The robotic controller may comprise at least one of (i) a force sensor for measuring force data indicative of a force exerted.on the at least one medical instrument, (ii) a pressure sensor for measuring a pressure value applied by a fluid inflation unit of the robotic controller for inflating the balloon catheter or for measuring the pressure value within a balloon catheter of the at least one medical instrument, and/or (iii) a volume sensor for measuring a volume value or relative volume of the balloon catheter.

This enables to provide more sensor data for the medical device controller and thus to compute more reliable control commands to achieve the target mechanical characteristic.

Further embodiments of the invention and improvement of the described embodiments will become apparent with the following description of the embodiments.

The invention is now described with reference to certain embodiments and figures which show:
Figure 1A: a schematic flow chart illustrative of the operation of the medical device controller for positioning at least one medical instrument,
Figure 1B: a schematic illustration of an embodiment of a medical system comprising a medical device controller with a control unit,
Figure 2: a schematic view of a robotic controller and medical instruments,
Figures 3A and 3B: CT imaging data of embodiments of a medical instrument assuming a target mechanical characteristic in the non-coronary cusp and the left ventricle,
Figure 4: a schematic illustration of an embodiment of the medical system comprising the medical device controller and a control unit connected to a display,
Figure 5A: a cross-sectional schematic view of the medical instruments for carrying out TAVI/TAVR comprising a pre-shaped guidewire, a heart valve prosthesis, and a delivery device,
Figure 5B: modified fluoroscopy imaging data showing a pre-shaped guidewire, heart valve prosthesis, and a delivery device, and
Figure 5C: fluoroscopy imaging data exemplary showing a heart valve prosthesis in the target mechanical characteristic in a fully deployed state.

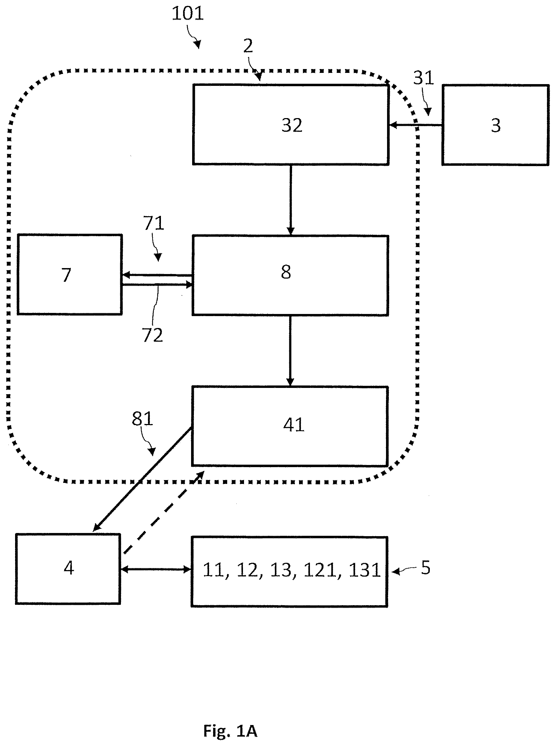

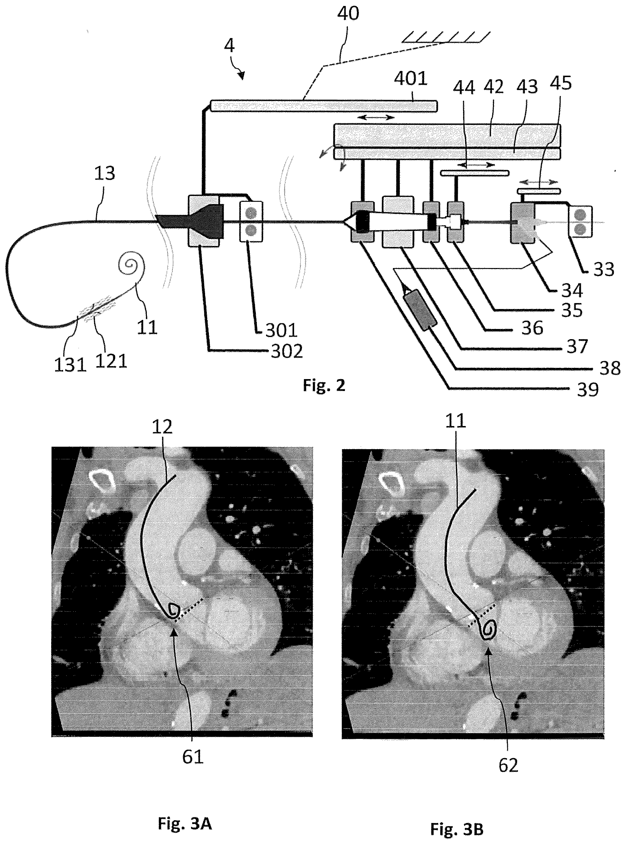

Figure 1A shows a schematic flow chart illustrative of the operation of the medical device controller 101 for positioning at least one medical instrument 11, 12, 13, 121, 131 (see Fig. 2) in proximity of an anatomic structure. The medical device controller comprises a control unit 2 which has an input interface 32, a processing unit 8, drive control feedback means 7, and a robotic interface 41.

The input interface 32 is at least unidirectionally connected to an imaging source 3, e.g. formed by a C-arm to receive fluoroscopy imaging data 31. The device controller 101 can also be configured to transmit control commands 81 to the imaging source 3, e.g. for transmitting control commands 81 indicative of an orientation/position of the C-arm to create imaging data of a specific location / at a selected perspective with respect to the patient. The imaging data 31 can be raw fluoroscopy images or pre-processed imaging data 31, e.g. normalized imaging data and/or segmented/annotated imaging data. Alternatively, the medical device controller 101 can be adapted for pre-processing the imaging data 31.

The processing unit 8 is adapted for receiving the imaging data 31 from the input interface 32. The processing unit 8 is also connected to a robotic interface 41 for at least unidirectional communication with a robotic controller 4. Moreover, the processing unit 8 is connected for bidirectional communication with the drive control feedback means 7. The control unit 2 has a feedback loop 71 established between the drive control feedback means 7 and the processing unit 8. The processing unit sends information about the current mechanical characteristic, e.g. mechanical state, of the medical instruments 11, 12, 13, 121, 131 based on the fluoroscopy imaging data to the feedback control means 7. The feedback control means-7 internally computes a control command needed to achieve the target mechanical characteristic, e.g. by reducing a distance between a current and target position. Subsequently, the feedback control means 7 transmits the control command back to the processing unit 8. Thereby, the feedback loop 71 enables to continuously update the control commands 81 in real-time based on feedback data 72 to move medical instruments 11, 12, 13, 121, 131 demonstrating a current mechanical characteristic 5 to achieve a target mechanical characteristic. The feedback data 72 are indicative of the current mechanical characteristic 5 with respect to the target mechanical characteristic. The relation between the current mechanical characteristic 5 and the target mechanical characteristic is continuously reevaluated based on the imaging data 31. The feedback data 72 are generated by the drive control feedback means 7.

The control commands 81 are continuously transmitted via the robotic interface 41, to a robotic controller 4 connected to the robotic interface 41. By operating the robotic controller 4, the medical instruments 11, 12, 13, 121, 131 are sequentially moved from demonstrating the current mechanical characteristic to achieving the target mechanical characteristic.

The robotic interface 41 and the robotic controller 4 may be connected in a bidirectional manner which allows the robotic controller 4 to send sensor data about its sensors, e.g. a position, a speed, a torque, and a temperature of the driver, a warning or an error message, or force/pressure/volume data.

The current mechanical characteristic 5 is determined via computer vision based on feature extraction of the imaging data. The target mechanical characteristic may be a predetermined characteristic which is computed by the processing unit 8 based on the patient specific anatomy for the different medical instruments 11, 12, 13, 121, 131. Alternatively, the target mechanical characteristic can be input and/or adjusted by a clinician via a user interface. The feedback loop to continuously update the control command 81 based on the feedback data can include sensor data of the robotic controller 4 or the medical instruments 11, 12, 13, 121, 131 which may be transmitted from the robotic controller 4 and which may be used for generating more targeted control commands 81.

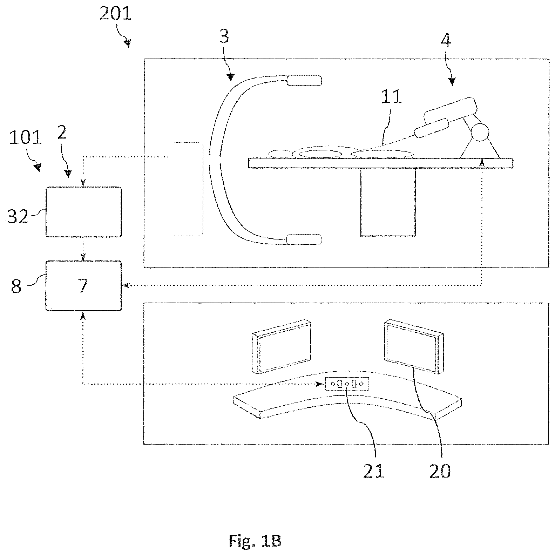

Figure 1B shows a schematic illustration of an embodiment of a medical system 201 comprising a medical device controller 101 with a control unit 2. The control unit 2 comprises an input interface 32, a processing unit 8 with drive control feedback means 7 as described above.

The input interface 32 is connected to an imaging source 3 which is formed by a C-arm fluoroscopy device in Fig. 1B. The input interface 32 is adapted for receiving two-dimensional or even three-dimensional imaging data, in particular two-dimensional fluoroscopy images from the imaging source 3.

The processing unit 8 is connected to the input interface 32 for receiving the imaging data and processing the imaging data. The processing unit 8 is connected via the robotic interface (not shown in Fig. 1B) to a robotic controller 4 which is adapted for manipulating a medical instrument 11, in particular a plurality of medical instruments (see Figs. 1A and 2). The processing unit 8 in Fig. 1B is adapted for bidirectional communication with the robotic controller 4.

The processing unit 8 is adapted for transmitting control commands which are determined by the drive control feedback means 7 to the robotic controller 4. Figure 1B shows that the drive control feedback means 7 determine the current mechanical characteristic of the medical instrument 11 based on the imaging data in real time.

The imaging data may be processed by the processing unit 8 via computer vision techniques such as classical transformations such as Canny Edge detection, Ridge detection, motion detection by background subtraction by using prior imaging data, color space conversion and color detection. The processing unit 8 may be adapted for using feature matching algorithms to find anatomic structures such as the native valve, a heart valve prosthesis, and/or the spine, in the imaging data. This may be achieved by using feature matching algorithms such as a "speeded up robust features algorithm" or an alternatively machine learning algorithm such as the Viola Jones algorithm or a convolutional neural network.

The target mechanical characteristic is preferably determined based on pre-operative planning by a clinician that may input the target mechanical characteristic into the medical device controller, e.g. via a user interface 21 connected to the processing unit 8.

Alternatively, the medical device controller 101 may be adapted to generate the target mechanical characteristic fully autonomously based on a selection of a specific heart valve prosthesis and the imaging data showing the heart valve prosthesis being advanced transfemorally to the aortic annulus. The target mechanical characteristic of a heart valve prosthesis can correspond to a configuration of the heart valve prosthesis which is advanced to the aortic annulus and was converted into a fully deployed state.

The drive control feedback means 7 generate feedback data based on the current mechanical characteristic and the target mechanical characteristic. A feedback loop which is established between the drive control feedback means 7 and the processing unit 8 is configured to continuously generate and send control commands based on the feedback loop to the robotic controller 4 to achieve the target mechanical characteristic.

In the specific embodiment the feedback loop of the medical device controller 101 is carried out by using an optimization algorithm which can be used for determining a plurality of control commands for different drivers of the robotic controller 4.

The robotic controller 4 is adapted for manipulating the medical instrument 11, e.g. a heart valve prosthesis, via drivers by rotating it, longitudinally advancing/retracting it, and tilting it, and deploying it, in particular by radially expanding the heart valve prosthesis via inflation of a balloon catheter or by removing a sheath for self-expansion.

The robotic controller 4 is adapted for sending sensor data, which are force data, deployment status data, data indicative of the current mechanical characteristic, cardiac activity data, respiratory activity data, and/or force/pressure/volume data to the processing unit 8. The feedback loop between the drive control feedback means 7 and the processing unit 8 is configured for further including the sensor data in the feedback loop to enhance the control commands to achieve the target mechanical characteristic.

The medical device controller 101 further comprises the user interface 21 which is adapted for receiving user instructions from a clinician. The user interface 21 is adapted to switch the medical device controller 101 between a feedback control mode and a tele-operative control mode. In the feedback control mode, the medical device controller 101 is adapted to send the control commands for the drivers of the robotic controller 4 in a fully autonomous manner in real-time based on the feedback loop. The feedback loop is closed such that no external inputs are required from the clinician, thereby avoiding a perception-cognition-action loop. However, by displaying the imaging data, or modified imaging data, e.g. including a graphic representation of the target mechanical characteristic, in particular the at least one medical instrument achieving the target mechanical characteristic, on displays 20 connected to the medical device controller 101, the procedure remains under full operator control and supervision. The clinician can always switch to the tele-operative control mode which allows for operator-enabled assistance.

In addition, the medical device controller 101 has an internal watchdog which prevents transmission of control commands if a malfunction or abnormal behaviour is detected. Such malfunctions/abnormal behaviours may be detected due to anatomical constraints, exceeding physical or safety limit constraints of the drivers to move the medical instruments, or exceeding pressure/volume constraints for deploying a balloon catheter during deployment of a heart valve prosthesis.

In the tele-operative control mode, the drivers of the robotic controller 4 are driven based on control commands of the medical device controller 101 which are generated based on input commands from the clinician. The input interface 21 may be formed by a control console, e.g. similar to a gaming console, or a customary joystick interface. The medical device controller 101 may comprise at least one user feedback element, e.g. a LED indicating the status and/or constraints or buttons, assessable by the clinician.

Figure 2 shows a schematic view of a robotic controller 4 and a plurality of medical instruments 11, 13, 121, and 131. The entire robotic controller 4 is positionable via a port fixation base 401 and a positioning arm 40, in particular a gravity compensating arm which is adjustable by a clinician, preferably in multiple degrees of freedom. The robotic controller 4 may be compatible with a commercially available system for deploying a heart valve prosthesis during TAVI/TAVR such as Edwards SAPIEN 3, e.g. as described in EP application No. 23315273.5 filed on July 7, 2023. The robotic controller described as cardiovascular navigation and actuation system in the above-mentioned patent application is hereby included by reference.

The robotic controller 4 actuates a medical instrument formed by a catheter shaft 13 which is positionable via a catheter feeding driver 301 which engages the catheter shaft 13 from two opposing sides with driven rollers. The robotic controller 4 further has a port receiving section 302 for coupling the medical instrument(s) 11, 13, 121, and 131 to the robotic controller 4.

One of the medical instruments is a balloon catheter 131 which is advanceable/retractable via a balloon shaft driver 34 connected to a balloon catheter base 45. The balloon catheter 131 may be inflatable via a balloon inflation driver 38, e.g. actuating a syringe. The robotic controller 4 further has a balloon locking element 35 and a balloon catheter locking base 44.

One of the medical instruments is a heart valve prosthesis 121 which may be arranged in a collapsed state over the balloon catheter 131 and can be expanded via the balloon catheter 131 upon inflation.

One of the medical instruments is a pre-shaped guidewire 11 for intraventricular placement, e.g. a SAFARI pacing guidewire. The pre-shaped guidewire 11 can be advanced/retracted via two actuated rollers of a guidewire driver 33.

The other medical instruments 131, 121, 13 may be advanced over this guidewire 11 after it was securely placed within the left ventricle.

A longitudinal position of the balloon catheter 131 relative to the catheter shaft 13 may be controlled with better precision than just translationally moving the balloon catheter 131 via a fine wheel driver 36. The fine wheel driver 36 is further adapted for modifying a stiffness of the balloon catheter 131.

A tilt of the catheter shaft 13 may be controlled by actuation of a flex wheel driver 39 for adjusting the tilt of the catheter shaft 13 and thus the position of the heart valve prosthesis 121. The fine wheel driver 36 and the flex wheel driver 39 are both connected to a handle insertion base 42 for longitudinal actuation and an actuation base 43 for rotational actuation of the wheels.

The previously described medical device controller is adapted to operate the drivers 301, 33, 34, 36, 39 via a plurality of control commands based on the feedback loop established between the drive control feedback means and the processing unit in real-time.

In particular the medical device controller can be adapted to operate the balloon locking element 35 to lock/unlock the position of the balloon catheter 131 or the inflation state of the balloon based. Alternatively, the balloon shaft driver 34 and/or the balloon inflation driver 38 may be locked/unlocked by the medical device controller.

The medical device controller can be adapted to advance/retract the balloon catheter 131 with respect to the handle base using linear actuation.

Based on the feedback loop, the medical device controller continuously updates the control commands for the different drivers to place the heart valve prosthesis 121 to achieve a target mechanical characteristic. The medical device controller and robotic controller 4 are preferably adapted for navigation of the medical instruments 11, 13, 131, 121 from a transfemoral access site into the femoral artery through the aorta to the aortic valve annulus. The medical device controller and robotic controller 4 may be adapted such that the guidewire 11 is navigated into the left ventricle to contact the left ventricle in the proximity of its apex.

After placing the guidewire 11, the other medical instruments 13, 121, 131 can be advanced over the guidewire 11 to move the heart valve prosthesis to achieve the target mechanical characteristic. The current mechanical characteristic of the medical instrument(s) with respect to the target mechanical characteristic is continuously used for generating feedback data via the drive control feedback means which are processed to generate real-time feedback data based on the feedback loop. The target mechanical characteristic of the heart valve prosthesis 121 corresponds to a deployed, i.e. radially expanded, state of the heart valve prosthesis 121 achieving a predetermined axial position in or in proximity of the annulus, i.e. a preferred implantation depth with respect to a position of the valve annulus, and a predefined central arrangement within the aortic annulus. The predefined central arrangement is defined in that the heart valve prosthesis 121 has a longitudinal axis which is essentially parallel with respect to the surrounding aortic annulus and centrally arranged with respect to the aortic annulus.

Figures 3A and 3B show CT imaging data of medical instruments 11, 12 achieving a target mechanical characteristic 61, 62.

Figure 3A shows a medical instrument which is formed by a pigtail catheter 12. The catheter 12 is positioned in the non-coronary cusp of the aortic valve annulus when achieving the first target mechanical characteristic 61. The catheter 12 is sized, shaped and placed for preventing potential blockage of the coronary ostia. When achieving the first target mechanical characteristic 61, the pigtail catheter 12 is arranged directly adjacent to the aortic valve annulus shown as a dashed line in Fig. 3A. The pigtail catheter 12 has a coiled pre-formed shape which enables atraumatic placement via the robotic controller and the medical device controller as previously described. The first target mechanical characteristic 61 relates to a pose, i.e. position and orientation, of the catheter 12.

Figure 3B shows a medical instrument formed by a pre-shaped SAFARI pacing guidewire 11 which is guided through the aortic annulus indicated by the dashed line and placed achieving a second target mechanical characteristic 62 in the left ventricle of the heart in proximity of the apex. The guidewire 11 has a pre-shaped coil tip or is deflectable to have a coiled tip which enables atraumatic, secure placement in the left ventricle. The second target mechanical characteristic 62 defines a pose of the guidewire 11.

The pose of the target mechanical characteristic 61, 62 includes a predefined curvature of the coiled tip of the medical instruments 11, 12 of Figs. 3A and 3B which may indicative reliable placement. The pose of the medical instruments 11, 12 in Figs. 3A and 3B is adapted for engaging the left ventricle/non-coronary cusp within safe force limits based on force data indicative of a force exerted on the guidewire 11 measured via the robotic controller which are included in the feedback loop. The control commands for achieving the target mechanical characteristics 61, 62 with the medical instruments 11, 12 are adapted to not exceed a predefined preset fixed force limit or a dynamic force limit.

The guidewire 11 and the catheter 12 shown in Figs. 3A and 3B can be controlled by control commands of the medical device controller to the robotic controller via two independent feedback loops established between drive control feedback means and a processing unit of the medical device controller.

The medical device controller is adapted to retain the guidewire 11 in Fig. 3B within the left ventricle of the heart reliably. The control command is continuously updated such that the guidewire maintains contact in vicinity of the apex of the left ventricle in a fully autonomous manner via the closed feedback loop. In a similar manner, the medical device controller is adapted for maintaining the catheter 12 in proximity of the non-coronary cusp. Thereby, the medical device controller is adapted for retaining the medical instruments 11, 12 to achieve the target mechanical characteristics 61, 62 shown in Figs. 3A and 3B.

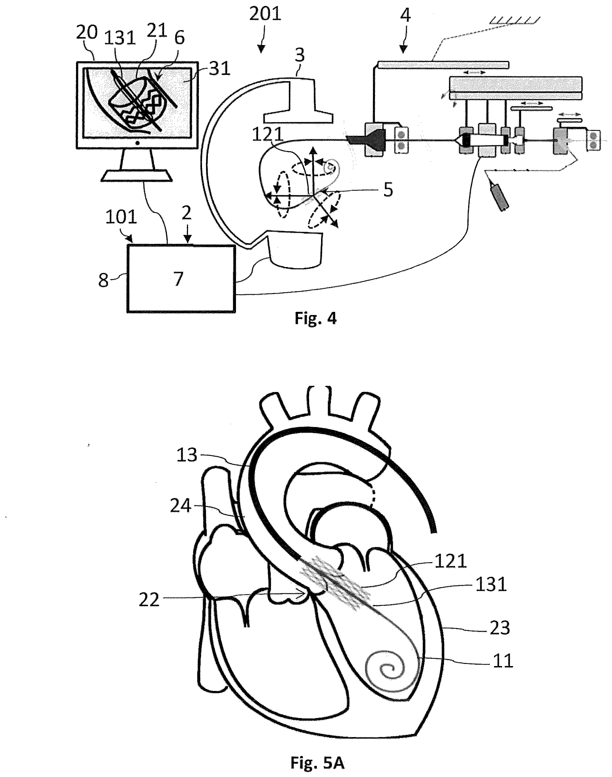

Figure 4 shows a schematic illustration of an embodiment of the medical system 201 comprising the medical device controller 101 and a control unit 2. The medical device controller 101 comprises the drive control feedback means 7, processing unit 8 and input interface for receiving imaging data as described above. The medical device controller 101 is connected to the imaging source 3 formed by the C-arm for acquiring fluoroscopy imaging data.

The motor controller 4 further sends sensor data, e.g. force data, a deployment status of the heart valve prosthesis 121, a current mechanical characteristic of a medical instrument, and/or cardiac/respiratory data, to the medical device controller 101 for improving the feedback cycle based on the sensor data.

The medical device controller 101 is connected to a display 20 to display the fluoroscopy imaging data 31 which have been augmented by the medical device controller 101 to show the medical instrument achieving a target mechanical characteristic 6. This display may also be overlayed over the medical instrument demonstrating a current mechanical characteristic 5 such that a clinician can supervise the procedure. Alternatively, the medical device controller 101 may be adapted to provide data for the display to display the medical instrument achieving the current and the target mechanical characteristic 5, 6 side by side on the display 20.

The C-arm 3 acquires the medical imaging data 31 of the medical instrument demonstrating the current mechanical characteristic 5, e.g. of the heart valve prosthesis 121 in a non-deployed state which was positioned in a central position of the heart valve annulus. Subsequently, in order to achieve the target mechanical characteristic 6, the medical device controller 101 continuously sends movement and deployment control commands to the robotic controller 4 to position and deploy the heart valve prosthesis 121 based on the feedback loop. These movement and deployment commands are determined based on a feedback loop between the drive control feedback means 7 and the processing unit 8 of the control unit.

The robotic controller 4 comprises a force sensor for measuring the forces exerted on the heart valve prosthesis 121 and/or the other medical instruments. During this procedure, the motor controller 4 repeatedly sends sensor data indicative of the deployment state of the heart valve prosthesis 121 and/or force data indicative of forces exerted on the heart valve prosthesis 121.

In addition, the imaging data 31 is used to generate feedback data indicative of the current mechanical characteristic 5 with respect to the target mechanical characteristic 6. This can be achieved by using a computer vision algorithm such as a feature matching algorithm which identifies the current mechanical characteristic 5 and the corresponding target mechanical characteristic 6 based on the known dimensions of the heart valve prosthesis.

The type of the heart valve prosthesis 121, and thus dimensions of the heart valve prosthesis 121, can be input to the medical device controller 101 by a clinician or be automatically detected by the processing unit 8.

The medical device controller 101 is adapted for deploying the heart valve prosthesis 121 in an automated inflation mode. In the automated inflation mode, the deployment control command is updated in an automated manner for inflating a balloon catheter 131 based on predetermined temporal pressure or volume function for applying pressure of supply fluid volume to the balloon catheter 131. In this manner, a non-linear deployment of the heart valve prosthesis 121 may be prevented and a fully autonomous deployment can be achieved.

The medical device controller 101 is further adapted to be switched to a user inflation mode in which the control command is updated based on a user input via an input interface 21 (see Fig. 1B). In the user inflation mode, the user can select a specific temporal pressure or volume function to apply pressure or supply a fluid volume to the balloon catheter 131 to deploy the heart valve prosthesis 121.

Figure 4 further schematically shows that the medical instruments, in particular the balloon catheter 131 over which the heart valve prosthesis 121 is arranged, may be actuatable in a plurality of degrees of freedom based on a movement control command of the medical device controller 101. In Fig. 4, the movement control command is configured for longitudinally, translationally moving the balloon catheter 131, rotating the balloon catheter 131, and tilting the shape of the balloon catheter 131. This enables placing the heart valve prosthesis 121 at a specific implantation depth, in a predefined rotational orientation, e.g. with respect to the coronary ostia, and tilting the balloon catheter 131 such that the heart valve prosthesis 121 is arranged in a central position of the valve annulus.

Figure 5A shows a cross-sectional schematic view of the medical instruments 121, 11, 131, 12 for carrying out TAVI/TAVR comprising a pre-shaped guidewire 11, a heart valve prosthesis 121, a delivery device formed by a catheter shaft 13, and a balloon catheter 131. The medical instruments 121, 11, 131, 12 are positioned achieving different target mechanical characteristics based on control commands of the medical device controller 101.

Each medical instrument 121, 11, 131, 12 may be driven via drivers of the robotic control unit and corresponding control commands independently from each other, e.g. based on a plurality of feedback loops or a common feedback loop for determining control commands. The target mechanical characteristic of the pre-shaped guidewire 11 is a target spatial configuration in the left ventricle 23 in proximity of the apex.

The target mechanical characteristic of the balloon catheter 131 is in a central position within the aortic annulus 21, essentially parallel to the vasculature in the annular region. This balloon catheter 131 may be longitudinally displaced, rotated, and/or tilted based on a movement control command to achieve this target mechanical characteristic.

The target mechanical characteristic of the catheter shaft 13 may be a placement in the aortic root 24 proximal from the aortic annulus 21.

The target mechanical characteristic of the heart valve prosthesis 121 may be a fully deployed state concentrically around a centerline of the aortic annulus at a predefined implantation depth (not shown in Fig. 5A, which schematically shows the valve position in a non-deployed state).

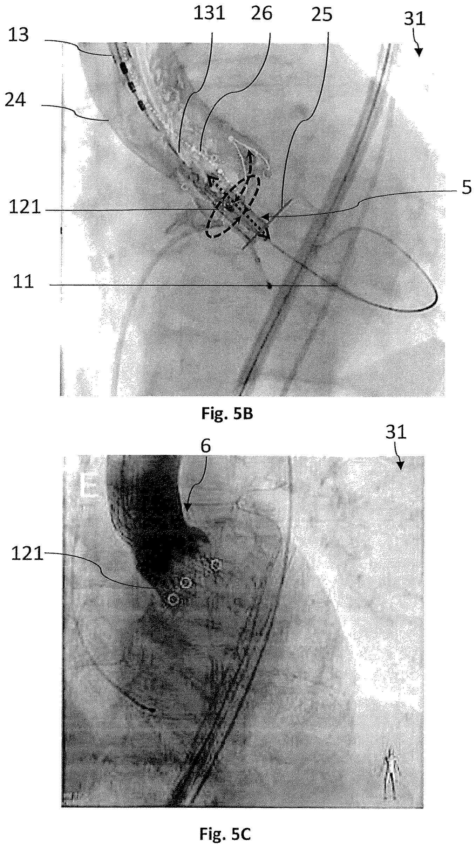

Figure 5B shows modified fluoroscopy imaging data 31 showing a pre-shaped guidewire 11, the heart valve prosthesis 121, and a delivery device formed by a catheter shaft 13 and a balloon catheter 131. The fluoroscopy imaging data 31 were modified by overlaying a model of aortic root 24 over the fluoroscopy imaging data 31. Moreover, a doted centerline which comprises a plurality of waypoints generated by a trajectory generation algorithm was computed via a control unit of a medical device controller and overlayed over the fluoroscopy imaging data 31. For optimal positioning of the heart valve prosthesis 121, the real-time movement commands position the heart valve prosthesis 121 by rotating it, longitudinally translating it, and/or tilting to be parallel to the centerline 26 and being arranged at a predetermined implantation depth 25. The movement of the balloon catheter 131 is exemplary shown in Fig. 5B to be movable in this manner by the plurality of dashed arrows.

The medical device controller in a feedback mode is adapted for fully autonomously arrange the balloon catheter 131 on which the heart valve prosthesis 121 is mounted parallel to the centerline 26. This may be achieved by at least one feedback loop, in particular a plurality of individual feedback loops, for positioning/orienting the medical instruments 11, 13, 121, 131 from demonstrating the current mechanical characteristics 5 shown in Fig. 5B to achieve the target mechanical characteristics.

In addition, the medical device controller is adapted to determine a position/orientation of both the coronary ostia with respect to the size and geometry of the heart valve prosthesis 121. To identify the position/orientation of the coronary ostia, fluoroscopy data in addition with a pre-operative model and/or CT data may be used. These data can be included in the feedback loop to modify the heart valve prosthesis achieving the target mechanical characteristic such that the coronary ostia are not obstructed.

Figure 5C shows fluoroscopy imaging data 31 exemplary showing a heart valve prosthesis 121 achieving a target mechanical characteristic 6 in a fully deployed state. The target mechanical characteristic 6 as shown Fig. 5C may be input by a user or determined by the medical device controller based on a current mechanical characteristic of a medical instrument, e.g. heart valve prosthesis 121, and the vasculature of the patient. The medical device controller can provide displaying data showing the medical instrument achieving the target mechanical characteristic 6, e.g. on a display, as an overlay of the fluoroscopy imaging data 31 shown in Fig. 5B showing the medical instrument demonstrating the current mechanical characteristic. Alternatively, the medical device controller may be adapted to display the medical instrument demonstrating the current and target mechanical characteristic as separate depictions to facilitate supervision of the medical device controller by a clinician.

## Claims

1. A medical device controller (101) for positioning at least one medical instrument (11, 12, 13) in proximity of an anatomic structure, in particular a valve annulus, wherein the medical device controller (101) comprises a control unit (2) which has:
- an input interface (32) connected or connectable to an imaging source (3) for receiving imaging data (31), in particular processed imaging data (31),
- a robotic interface (41) connected or connectable to a robotic controller (4) for transmitting control commands (81) to the robotic controller (4),
- a processing unit (8) adapted for receiving imaging data (31) via the input interface (32) and adapted for transmitting at least one control command (81) via the robotic interface (41) to the robotic controller (4) for adjusting at least one medical instrument (11, 12, 13) to achieve a target mechanical characteristic (6), and
- drive control feedback means (7) configured for generating feedback data (72) indicative of a current mechanical characteristic (5) of the at least one medical instrument (11, 12, 13) with respect to the target mechanical characteristic (6) and transmitting the feedback data (72) to the processing unit (8),
wherein the control unit (2) has a feedback loop (71) established between the drive control feedback means (7) and the processing unit (8) which is configured to continuously update the at least one control command (81) in real-time based on the feedback data (72) to achieve the target mechanical characteristic (6).

2. The medical device controller (101) according to claim 1, wherein the drive control feedback means (7) are configured for generating:
- first feedback data indicative of a first current mechanical characteristic (5) of a first medical instrument (11), in particular a guidewire, with respect to a first target mechanical characteristic, and
wherein the control unit (2) has
- a first feedback loop established between the drive control feedback means (7) and the processing unit (8) which is configured to continuously update a first control command in real-time based on the first feedback data to achieve the first target mechanical characteristic of the first medical instrument (11).

3. The medical device controller (101) according to one of the preceding claims, wherein the drive control feedback means (7) are configured for generating:
- second feedback data indicative of a second current mechanical characteristic of a second medical instrument (12), in particular a heart valve prosthesis (121) or a delivery device (13), with respect to a second target mechanical characteristic, wherein the second current mechanical characteristic is preferably different from the first current mechanical characteristic,
wherein the control unit (2) has
- a second feedback loop established between the drive control feedback means (7) and the processing unit (8) which is configured to continuously update a second control command to achieve the second target mechanical characteristic of the second medical instrument (12).

4. The medical device controller (101) according to one of the preceding claims, wherein the drive control feedback means (7) are configured for generating the feedback data, based on the imaging data (31), in particular fluoroscopy or computer tomography imaging data (31), from the imaging source (3) indicative of the current mechanical characteristic (5) of the at least one medical instrument (11, 12, 13) and the target mechanical characteristic (6) of the at least one medical instrument (11, 12, 13), in particular with respect to an anatomic structure of the patient.

5. The medical device controller (101) according to one of the preceding claims, wherein the feedback loop is configured to:
- Determine a position of at least one of the coronary ostium, in particular both coronary ostia, and
- Determine if the heart valve prosthesis achieving the target mechanical characteristic would obstruct the coronary ostium, and
- if the coronary ostium would be obstructed, modify the target mechanical characteristic such that the coronary ostium is not obstructed.

6. The medical device controller (101) according to one of the preceding claims, wherein the at least one control command comprises a movement control command for at least one driver of the robotic controller (4):
- for longitudinal translation of the at least one medical instrument (11, 12, 13),
- for rotating the at least one medical instrument (11, 12, 13) around a longitudinal axis of the at least one medical instrument (11, 12, 13),
- for tilting the at least one medical instrument (11, 12, 13) with respect to the longitudinal axis, and/or
- changing a shape of the at least one medical instrument (11, 12, 13).

7. The medical device controller (101) according to one of the preceding claims, wherein the at least one control command comprises a deployment control command for deploying the medical instrument (11, 12, 13) formed by a heart valve prosthesis (121), wherein
the drive control feedback means (7) are configured for generating feedback data indicative of a deployment status of the heart valve prosthesis (121), in particular feedback data based on at least one of:
- a deployment status indicative of a self-expanding heart valve prosthesis,
- a pressure value, preferably an input pressure value or a pressure value measured in a balloon catheter for deploying the heart valve prosthesis,
- a volume value of a balloon catheter for deploying the heart valve prosthesis, and
- a relative volume with respect to a maximum volume of the balloon catheter for deploying the heart valve prosthesis.

8. The medical device controller (101) according to claims 6 and 7, wherein the feedback loop of the control unit (2) is configured to continuously update the movement control command, in particular for longitudinal translation, and the deployment control command in a synchronized manner during deployment of the heart valve prosthesis.

9. The medical device controller (101) according to one of the preceding claims, wherein the robotic interface is adapted for receiving sensor data, in particular from the robotic controller (4), and the drive control feedback means (7) is adapted for generating the feedback data based on the received sensor data.

10. The medical device controller (101) according to claim 9, wherein the sensor data are selected from at least one of
- force data indicative of a force exerted on the at least one medical instrument (11, 12, 13) from the robotic controller (4),
- data indicative of a deployment status of the heart valve prosthesis (121) from the robotic controller (4),
- data indicative of a current mechanical characteristic (5) of the at least one medical instrument (11, 12, 13), and/or
- cardiac activity data and/or respiratory activity data indicative of a cardiac rhythm and/or respiratory rhythm of the patient.

11. The medical device controller (101) according to one of the preceding claims, wherein the drive control feedback means (7) are configured for generating feedback data based on a plurality of input data from different sources, in particular selected from the imaging data (31) of the imaging source (3) of claim 4 and from the sensor data of claim 9 from the robotic controller (4), wherein
the drive control feedback means (7) is configured for applying a sensor fusion algorithm, in particular a Kalman Filter algorithm, to the plurality of input data to generate the feedback data.

12. The medical device controller (101) according to one of the preceding claims, wherein the control unit (2) is adapted for determining, if the control command to achieve the target mechanical characteristic would result in a motor controller being controlled such as to exceed a force limit of the at least one medical instrument (11, 12, 13) interacting with the anatomy of the patient, wherein
if exceeding the force limit was detected, the control unit is configured for adapting the control command such that the force limit is not exceeded, wherein
the force limit is preferably one of
- (i) a preset fixed force limit,
- (ii) a dynamic force limit determined based on the current mechanical characteristic (5) of the at least one medical instrument (11, 12, 13) with respect to the anatomic structure of the patient, in particular the left ventricle, and/or
- (iii) a dynamic force limit determined based on an angle of incidence of a longitudinal axis of the medical instrument (11, 12, 13) interacting with the anatomic structure of the patient.

13. The medical device controller (101) according to one of the preceding claims, wherein the current and/or target mechanical characteristic (5, 6) of the at least one medical instrument (11, 12, 13) with respect to a patient anatomy is selected from at least one of:
- A position, shape, and/or orientation of a first medical instrument formed by a guidewire (11) or catheter with respect to a native valve annulus or a native apex of the left ventricle,
- A position, shape, and/or orientation of a second medical instrument (12) formed by a heart valve prosthesis () with respect to a native valve annulus,
- A deployment status of a medical instrument (12), in particular a heart valve prosthesis (121) and/or a balloon catheter (131).

14. The medical device controller (101) according to one of the preceding claims, wherein the control unit (2) is adapted for generating a planned trajectory of the at least one medical instrument (11, 12, 13), in particular a delivery device (13), the planned trajectory extending from the at least one medical instrument (11, 12, 13) demonstrating the current mechanical characteristic (5) to the at least one medical instrument achieving the target mechanical characteristic (6), wherein the planned trajectory is generated via a trajectory generation algorithm based on a model of the anatomic structure of the patient received via the in-put interface, wherein
the model of the anatomic structure is preferably a pre-operative model of the anatomic structure of the patient, in particular a CT model of the aorta.

15. The medical device controller (101) according to claim 14, wherein the trajectory generation algorithm is selected from at least one of a Dijkstra algorithm, a look-ahead tree search algorithm, a probabilistic roadmap, rapidly exploring random trees, and a centreline algorithm, wherein the trajectory generation algorithm preferably is adapted to generate a plurality of waypoints.

16. The medical device controller (101) according to claim 15, wherein the control unit (2) is adapted for determining or receiving a centreline with respect to an aorta wall of the aorta model, wherein the control unit (2) is adapted for generating the planned trajectory by minimizing the distance of the medical instrument (11, 12, 13) to the centreline and/or by following the plurality of waypoints.

17. The medical device controller (101) according to one of the preceding claims, wherein the medical device controller (101) is adapted for displaying the imaging data of the imaging source (3) and in particular an information indicative of a relation between the current mechanical characteristic (5) and the target mechanical characteristic (6).

18. The medical device controller (101) according to one of the preceding claims, wherein the medical device controller (101) is operable at least in:
- a feedback control mode in which the feedback loop is configured to continuously update the at least one control command in real-time based on the feedback data to achieve the target mechanical characteristic, and
- a tele-operative control mode, in which the device controller (101) is adapted to receive input commands from a user and generate the control command, in particular the movement and/or deployment control command, via the control unit (2) based on the input commands instead of the feedback loop, wherein
the medical device controller (101) has a user interface (21), in particular a graphical user interface, adapted to receive user instructions to switch between the feedback control mode and the tele-operative control mode.

19. The medical device controller (101) according to one of the preceding claims, wherein the medical device controller (101) is operable in an automated inflation mode and/or a user input inflation mode:
- wherein in the automated inflation mode, the deployment control command is updated in an automated manner by at least one of:
○ A predetermined pressure or volume limit for a safe operation of the balloon catheter (131),
○ A predetermined inflation time of the balloon catheter (131) to achieve the deployment of the heart valve prosthesis, and
○ A predetermined temporal pressure or volume function to apply pressure or supply a fluid volume to the balloon catheter (131),
- wherein in the user input inflation mode, the deployment control command is updated via a user input via a user interface, in particular a graphical user interface, wherein the user input is indicative of at least one of:
○ A pressure or volume limit for a safe operation of the balloon catheter (131),
○ An inflation time of the balloon catheter (131) to achieve the deployment of the heart valve prosthesis, and
○ A temporal pressure or volume function to apply pressure or supply a fluid volume to the balloon catheter (131).

20. The medical device controller (101) according to one of the preceding claims, wherein the control unit (2) is adapted to
- fully autonomously update the control command to retain the at least one medical instrument (11, 12, 13) in a specific anatomic structure, optionally selected from:
○ the left ventricle and/or the aortic annulus, preferably maintaining contact with or in the vicinity of the apex of the left ventricle, wherein the at least one medical instrument is preferably formed by a guidewire, or
○ the non-coronary cusp, or the medical instrument achieving the target mechanical characteristic, wherein the at least one medical instrument (11, 12, 13) is preferably formed by a catheter,
and/or
- to provide a warning signal to a user, in particular on a graphical user interface (21), if the medical instrument (11, 12, 13) is not retained in the specific anatomic structure.

21. The medical device controller (101) according to one of the preceding claims, wherein the at least one feedback loop, in particular all feedback loops, established between the drive control feedback means (7) and the processing unit (8) for continuously updating the control command is based on at least one of:
- A linear feedback control mode,
- A proportional-integral-derivative feedback control mode,
- An optimization-based control mode based on the feedback data and a set of constraints, in particular defined by (i) the anatomic structure constraints, (ii) operational constraints, and/or (iii) physical or safety limit constraints, and/or
- A neural network feedback control mode.

22. The medical device controller (101) according to claim 21, wherein the feedback loop established between the drive control feedback means (7) and the processing unit (8) is configured for continuously updating at least a first control command and a second control command for different drivers of the robotic controller (4) to achieve the target mechanical characteristic, wherein the feedback loop includes the steps of computing an optimization algorithm which comprises:
- relating the actuation of different drivers to adjust the at least one medical instrument demonstrating the current mechanical characteristic to achieve the target mechanical characteristic,
- determining at least a first and second control command for the at least two different drivers which minimize a difference between the current mechanical characteristic and the target mechanical characteristic.

23. A medical system (201) comprising the medical device controller (101) according to one of the preceding claims and a robotic controller (4) having a plurality of drivers for moving the at least one medical instrument (11, 12, 13).

24. The medical system (201) according to claim 23, wherein the robotic controller (4) comprises at least one of:
- a force sensor for measuring force data indicative of a force exerted on the at least one medical instrument (11, 12, 13),
- a pressure sensor for measuring a pressure value applied by a fluid inflation unit of the robotic controller (4) for inflating the balloon catheter (131), or
measuring the pressure value within a balloon catheter (131) of the at least one medical instrument (11, 12, 13), and/or
- a volume sensor for measuring a volume value or relative volume of the balloon catheter (131).
